# EUROPEAN PATENT APPLICATION

(11) **EP 3 725 941 A1**
(43) Date of publication of application: **21.10.2020**
(21) Application number: 17928951.7
(22) Date of filing: 20.10.2017
(51) Int. Cl.: D06M 13/00, A61L 9/01

(54) **PERFUME COMPOSITION FOR INHIBITING ODOR**

(71) Applicant: LG Household & Health Care Ltd., Seoul 03184 (KR)
(72) Inventor: KIM, Hoo-Deok, Anyang-si Gyeonggi-do 13977 (KR); HA, Jinsu, Anyang-si Gyeonggi-do 13977 (KR)
(74) Representative: Krauss, Jan
(86) International application number: PCT/KR2017/011685
(87) International publication number: WO 2019/078385

(57) **Abstract**

The present invention relates to a fragrance composition for suppressing malodor, comprising an alcohol fragrance, a lactone fragrance, or both, and more specifically, to a fragrance composition that can suppress malodor and unpleasant odor that may be generated by the human body, or clothes or environments being in contact with the human body. The fragrance composition of the present invention was completed by applying a single fragrance of an alcohol fragrance or lactone fragrance, or a mixture of both, thus being able to more effectively act against malodor while providing a more luxurious and excellent aromatic property compared to the conventional fragrances with a rather simple aromatic property.

## Description

### [Technical Field]

The present invention relates to a fragrance composition which contains an alcohol fragrance, a lactone fragrance, or a both, and more specifically, to a fragrance composition which is able to mask unpleasant odors including malodors derived from the human body, or fabrics (*e.g*., clothes) being in direct contact with the human body.

### [Background Art]

In general, household goods and cosmetics are used to maintain a hygienic and healthy life. Further, it is common to blend various raw materials and materials in order to enhance the functions and efficacy of these products. In the case of wash-off products, malodor sources caused by contamination may be removed by applying surfactants with various detergency levels. It is also a common practice to add various additional raw materials to impart functions and characters on these products. However, since the presence of any malodor in a product, even at a minimum level, can be considered as a serious defect, it is important to remove such a defect from products. Additionally, the degree of elimination of a malodor and an unpleasant odor generated by a product can directly affect the purchasing power of the product and may create an adverse effect to reduce its after-use satisfaction. Therefore, many companies have been making efforts to control these bad odors.

A conventional method generally used to reduce any malodor or unpleasant odor of a product is to increase the fragrance rate of a product. However, an excessively high fragrance rate of a given product has limitations in that it may raise the issue of stability of the product, may generate an altered odor over time, and may increase the cost of the product.

Another method to reduce the malodor or unpleasant odor of a product is to control these offensive odors by utilizing the diffusing character of a fragrance. One of the methods of masking these offensive odors is to increase the sillage of a fragrance, and for this purpose, it is preferable to use a fragrance which includes a high percentage of raw materials with a boiling point of 250°C or higher and a ClogP (a solubility index) value of 3 or higher (US Pat. No. 6,086,903). Fragrance sensation is a characteristic feature associated with a fragrance being elicited by its volatilization due to a vapor pressure. Since the vapor pressure is inversely proportional to the boiling point of a material, the sillage of a fragrance increases as the boiling point of the fragrance becomes higher. In contrast, a fragrance material with high volatility due to a low boiling point is characterized in that it volatizes well, and thus, it is possible to instantly mask malodors. However such a fragrance material has limitations in that the malodor-masking effect being felt after use or during storage of the product does not last long. Further, such a fragrance material also has limitations in that the fragrance character is simple and is thus unable to meet the customers' requests for implementation and differentiation of an excellent emotional quality.

As such, since fragrances have a very important effect on the emotional quality for consumers, various methods have been attempted to remove malodors or unpleasant odors generated via various routes. Nevertheless, it is still very difficult to prepare fragrances having both a desired aroma and an intensity that meets the customers' emotional needs.

### [Disclosure]

### [Technical Problem]

Under the circumstances, in order to solve the problem in that malodors and unpleasant odors generated in the human body are accumulated in the clothes, *etc*., the present inventors have conducted studies to find an optimal combination of fragrances among the fragrance ingredients thereof that can effectively mask the malodors. As a result, the present inventors have confirmed that when various alcohol fragrances or lactone fragrances are contained in a fragrance composition, it is possible for the fragrance to mask malodors and evoke the emotional quality of a luxurious fragrance away from a monotonous fragrance. In particular, the present inventors have confirmed that the alcohol fragrance can be comprised of various aroma series beyond the limits of the monotonous aroma of conventional fragrances, which had been applied for the purpose of masking, thereby completing the present invention. That is, the present inventors have confirmed that alcohol-based fragrances have the most excellent masking effect, and additionally, that the masking effect of alcohol-based fragrances is enhanced when used together with lactone-based fragrances. Accordingly, the present inventors have completed the preparation of a fragrance composition by utilization of the above discoveries, and the preparation of home care products and personal care products which include the fragrance composition.

### [Technical Solution]

An object of the present invention is to provide a fragrance composition for suppressing malodors, containing an alcohol fragrance, a lactone fragrance, or both.

Another object of the present invention is to provide a home care product for suppressing malodors, including the fragrance composition.

Still another object of the present invention is to provide a personal care product for suppressing malodors, including the fragrance composition.

Still another object of the present invention is to provide a method for suppressing malodors, including applying the fragrance composition to a malodor source.

Still another object of the present invention is to provide a use of the fragrance composition for suppressing malodors using the fragrance composition.

### [Advantageous Effects]

The fragrance composition of the present invention, by containing an alcohol fragrance or a lactone fragrance therein, can suppress various kinds of malodors and unpleasant odors generated from the human body, clothes, and environments, and induce a luxurious aroma, and is thus expected to have an effect of enhancing the property of sillage.

### [Best Mode for Carrying Out the Invention]

In order to solve the above problems and achieve the purposes of the present invention, an aspect of the present invention provides a fragrance composition, which contains an alcohol fragrance, a lactone fragrance, or both.

The present invention provides a fragrance composition, which contains an alcohol fragrance, a lactone fragrance, or both.

Additionally, the present invention provides a use of the fragrance composition, which contains an alcohol fragrance, a lactone fragrance, or both.

As used herein, the term "malodor" refers to an off-odor, an altered odor, an unpleasant odor, or an unpleasant off-odor that deteriorates the quality of a product, which are generated secondarily by chemical changes in food ingredients and an introduction of foreign materials from the outside. All of the odors caused by the incorporation of other materials, such as a contamination odor, an odor associated with a decrease in freshness, a rancid odor, an oxidized odor, a sunlight odor, and a heated odor, may be included in the category of the malodor. Examples of the odors caused by contamination may include a moldy odor in a wet and humid basement, an unpleasant odor in wet long-neglected laundry, *etc.* It is assumed that methyl oxide plays a major role in the generation of these odors by reacting with hydrogen sulfide to produce 4-methyl-4-mercaptopentan 2-one. These odors appear in meat, dairy products, fruits, fish food products, *etc.*

When the meat of birds bred with a feed containing fish flour and fish oil is cooked, it produces a fishy smell. Meanwhile, when the meat of birds, which has been left unattended without removing the bones after slaughter, is cooked, it produces malodors of organ smells.

As one of malodors of oils, there is a reversed odor.

When soybean oil, corn oil, *etc.* are stored, they generate a green and fishy smell at an early stage rather than a rancid odor, where *cis* 3-hexenal and other 2-pentyl furans are known as the main compounds responsible for the reversed odor. Hexanal is well known as a source of the rancid odor associated with chicken fries and potato fries, and is known as an indicator of the unpleasant off odor of an oxidative flavor. There are two odors generated by milk: an oxidized odor caused by fatty acids and sunlight odor associated with milk protein. When milk is exposed to sunlight, it produces an unpleasant odor, which is generated when cabbages are boiled or hairs are burnt. This is sunlight odor caused by a reaction between methionine and riboflavin. When beer is placed in the sunlight, it generates a malodor similar to fox urine. This is thought to occur as the main component of hop, humulone, is transformed into isohumulone by heating, and part of it is converted to prenylmercaptan by the sunlight. In an embodiment of the present invention, isobutyric acid, isovaleric acid, caproic acid, caprylic acid, 2-nonenal, 6-heptenoic acid, and pelargonic acid were used as a malodor-causing source (Experimental Example 4 and Table 8).

As used herein, the term "malodor suppression" means that a malodor is removed or masked, and as a result of sensory evaluation, it is determined that there is no malodor or the malodor is weak. Methods of suppressing a malodor may include deodorization, masking, *etc*., but the methods are not limited thereto.

As used herein, the term "alcohol fragrance" refers to a compound having -OH (alcohol group) as a functional group, and typically, at least one alcohol fragrance ingredient may be included in a combined fragrance that is used to mask the malodors of household goods, cosmetic products, *etc.*

The combined fragrance may include at least one selected from the group consisting of abienol, ambrinol, amyl vinyl carbinol, anisyl alcohol, Bacdanol, Sandalrom, benzyl alcohol, Bornafix, Brahmanol, carbinol muguet, cedrol, cinnamyl alcohol, citronellol, Corps Eglantine, Dartanol, decanol, Delphol, dihydro myrcenol, dihydroterpineol, dimethyl octanol, Dimetol, Dimyrcetol, dimethyl benzyl carbinol, Ebanol, ethyl linalool, eugenol, farnesol, Firsantol, Florol, Florosa, geraniol, Grisalva, hexanol, *cis*-3-hexenol, Hydroxyambran, hydroxyol, isoamyl alcohol, isopulegol, Javanol, linalool, Lindenol, Madranol, Majantol, Mayol (*cis*-muguet shiseol), Mefrosol, Meijiff, 1-menthol, 4-methyl guaiacol, Mugetanol, muguesia, muguet alcohol, mysantol, nerol, nerolidol, Norlimbanol, nonanol, Pamplefleur, patchouli alcohol, peomosa, Phenoxanol, phenoxyethyl alcohol, phenyl ethyl alcohol, phenylpropyl alcohol, Polysantol, prenol, rosalva, Rosaphen, Sandalore, Sandela, Sandranol, Sanjinol, Santaliff, Silvial, styrallyl alcohol, α-terpineol, β- terpineol, 4-terpinenol, Terranol, tetrahydrolinalool, Tetrahydro Muguol, tetrahydro myrcenol, thymol, Timberol, Ultravanil, Undecavertol, santol pentenol, *etc.;* and more preferably, at least one selected from the group consisting of decanol, ambrinol, dihydro myrcenol, nerol, Pamplefleur, geraniol, linalool, phenyl ethyl alcohol, rosalva, tetrahydrolinalool, α-terpineol, styrallyl alcohol, *cis*-3-hexenol, anisyl alcohol, Dimetol, cinnamyl alcohol, cedrol, patchouli alcohol, and Timberol.

As used herein, the term "lactone fragrance" is a generic term for a heterocyclic carboxylic acid ester having a -COO- bond in the molecule, which has properties similar to ether, and is called β-lactone, γ-lactone, and δ-lactone according to the size of its ring. At least one lactone fragrance ingredient may be included in a combined fragrance that is used to mask the malodors of household goods, cosmetic products, *etc.* The combined fragrance may include α-angelica lactone, γ-butyrolactone, coumarin, cycletene, γ-decalactone, δ-decalactone, γ-dodecalactone, ethyl fenugreek lactone, fenugreek lactone, γ-hexalactone, *cis*-jasmone lactone, juniper lactone, massoia lactone, γ-octalactone, γ-nonalactone, γ-undecalactone, whiskey lactone, *etc*., in which microcyclic lactones may include at least one selected from the group consisting of ambrettolide, ethylene brassylate, Exaltolide, isoambrettolide, musk lactone, *etc.;* and more preferably, may include at least one selected from the group consisting of γ-undecalactone, *cis*-jasmone lactone, and musk lactone.

The fragrance composition of the present invention may contain at least one selected from the group consisting of alcohol fragrances and may contain at least one selected from the group consisting of lactone fragrances.

In Table 1 and Table 2, fragrance ingredients that may be contained in the fragrance composition are indicated, however, the fragrance ingredients that may be contained in the fragrance composition of the present invention is not limited to those ingredients shown in Tables 1 and 2 below.

**[Table 1] Major alcohol fragrances**

| Name of Fragrance Subtance | Scent | clogP Value |
|---|---|---|
| Decanol | aldehydic | 4.57 |
| Nonanol | aldehydic | 3.77 |
| Abienol | amber | 6.40 |
| Ambrinol | amber | 3.84 |
| Bornafix | amber | 3.49 |
| Grisalva | amber | 6.58 |
| Hydroxyambran | amber | 6.30 |
| 4-Terpinenol | citrus-series | 3.26 |
| Dihydro Myrcenol | citrus-series | 2.99 |
| Dimyrcetol | citrus-series | 4.20 |
| Nerol | citrus-series | 2.70 |
| Pamplefleur | citrus-series | 3.01 |
| Tetrahydro Myrcenol | citrus-series | 3.60 |
| Benzyl Alcohol | floral | 1.10 |
| Carbinol Muguet | floral | 2.93 |
| Citronellol | floral | 3.10 |
| Corps Eglantine | floral | 2.37 |
| Delphol | floral | 3.68 |
| Dihydro Terpineol | floral | 3.22 |
| Dimethyl Benzyl Carbinol | floral | 2.27 |
| Dimethyl Octanol | floral | 3.85 |
| Ethyl Linalool | floral | 3.30 |
| Farnesol | floral | 4.80 |
| Florol | floral | 1.50 |
| Florosa | floral | 2.00 |
| Geraniol | floral | 2.60 |
| Hydroxyol | floral | 2.54 |
| Linalool | floral | 2.97 |
| Maj antol | floral | 3.48 |
| Mayol (*cis*-Muguet Shiseol) | floral | 3.45 |
| Mefrosol | floral | 3.20 |
| Meijiff | floral | 3.45 |
| Mugetanol | floral | 3.54 |
| Muguesia | floral | 2.89 |
| Muguet Alcohol | floral | 2.93 |
| Nerolidol | floral | 4.68 |
| Peomosa | floral | 2.11 |
| Phenoxanol | floral | 2.7 |
| Phenoxy ethyl Alcohol | floral | 1.16 |
| Phenylethyl Alcohol | floral | 1.36 |
| Phenyl Hexanol (Phenoxanol) | floral | 3.00 |
| Phenylpropyl Alcohol | floral | 1.88 |
| Rosalva | floral | 3.70 |
| Rosaphen | floral | 3.46 |
| Silvial | floral | 4.00 |
| Tetrahydrolinalool | floral | 3.49 |
| Tetrahydro Muguol | floral | 3.60 |
| Undecavertol | floral | 3.90 |
| α- Terpineol | floral | 2.60 |
| β- Terpineol | floral | 2.837 |
| Lindenol | fresh | 2.60 |
| Isoamyl Alcohol | fruity | 1.16 |
| Prenol | fruity | 1.055 |
| Styrallyl Alcohol | fruity | 1.41 |
| Ultravanil | gourmand | 2.20 |
| *cis*-3-Hexenol | green-series | 1.70 |
| Hexenol | green-series | 2.03 |
| Amyl Vinyl Carbinol | herbal | 2.40 |
| Anisyl Alcohol | herbal | 1.00 |
| Dimetol | herbal | 3.00 |
| Isopulegol | herbal | 2.72 |
| 1-Menthol | herbal | 3.40 |
| Thymol | herbal | 3.30 |
| 4-Methyl Guaiacol | spicy-series | 1.93 |
| Cinnamyl Alcohol | spicy-series | 1.95 |
| Eugenol | spicy-series | 2.27 |
| Bacdanol (Sandalrom) | woody-series | 4.30 |
| Brahmanol | woody-series | 4.73 |
| Cedrol | woody-series | 3.90 |
| Dartanol | woody-series | 4.44 |
| Ebanol | woody-series | 4.90 |
| Firsantol | woody-series | 4.43 |
| Javanol | woody-series | 4.80 |
| Madranol | woody-series | 5.31 |
| Mysantol | woody-series | 4.7 |
| Norlimbanol | woody-series | 5.42 |
| Patchouli Alcohol | woody-series | 4.48 |
| Polysantol | woody-series | 4.70 |
| Sandalore | woody-series | 4.70 |
| Sandela | woody-series | 5.60 |
| Sandranol | woody-series | 5.14 |
| Sanjinol | woody-series | 4.30 |
| Santaliff | woody-series | 4.65 |
| Santol Pentenol | woody-series | 4.778 |
| Terranol | woody-series | - |
| Timberol | woody-series | 5.80 |

**[Table 2] Major lactone fragrances**

| Name of Fragrance Subtance | Scent | clogP Value |
|---|---|---|
| γ- Decalactone | fruity | 2.72 |
| γ- Dodecalactone | fruity | 3.47 |
| γ-Nonalactone | fruity | 1.942 |
| γ-Undecalactone | fruity | 2.961 |
| γ-Octalactone | fruity | |
| δ- Decalactone | fruity | 2.469 |
| *cis*-Jasmone Lactone | gourmand | 2.117 |
| Coumarine | gourmand | 1.39 |
| Cycletene | gourmand | 0.26 |
| α-Angelica Lactone | gourmand | 0.236 |
| Ethyl Fenugreek Lactone | gourmand | 0.213 |
| Fenugreek Lactone | gourmand | -0.296 |
| Massoia Lactone | gourmand | 2.419 |
| Whiskey Lactone | gourmand | 1.968 |
| γ-Butyrolactone | gourmand | -0.64 |
| γ-Hexal actone | gourmand | 0.413 |
| Ambrettolide | musky | 5.429 |
| Ethylene Brassylate | musky | 2.773 |
| Exaltolide | musky | 6.10 |
| Isoambrettolide | musky | 6.51 |
| Juniper Lactone | musky | 6.65 |
| Musk Lactone | musky | 3.531 |

The fragrance composition of the present invention is especially effective in masking the malodors originating from the human body, clothes, environments, *etc*., and at least one selected from the group of alcohol fragrances described in Table 1 may be used, but is not limited thereto.

In order to enhance the excellence of fragrance quality and to strengthen the malodor masking ability, at least one selected from the group of lactone fragrances described in Table 2 may be used, but is not limited thereto.

Additionally, the present invention can provide a fragrance composition in which an alcohol fragrance is contained in an amount of 10 wt% to 65 wt% relative to the total weight of the composition. Specifically, the fragrance composition may contain an alcohol fragrance in an amount of 15 wt% to 65 wt%. More specifically, the fragrance composition may contain an alcohol fragrance in an amount of 20 wt% to 65 wt%, 25 wt% to 65 wt%, or 30 wt% to 65 wt%. Even more specifically, the fragrance composition may contain an alcohol fragrance in an amount of 35 wt% to 65 wt%, 40 wt% to 65wt%, or 45 wt% to 65 wt%. Most specifically, the fragrance composition may contain an alcohol fragrance in an amount of 50 wt% to 65 wt%, 55 wt% to 65 wt%, or 60 wt% to 65 wt%. When the alcohol fragrance is contained less than 10 wt% relative to the total amount of the fragrance, the malodor masking effect is not enough, whereas when the alcohol fragrance is contained greater than 65 wt%, the preference to the fragrance is reduced.

Additionally, the present invention can provide a fragrance composition in which a lactone fragrance is contained in an amount of 5 wt% to 65 wt% relative to the total weight of the composition. Specifically, the fragrance composition may contain a lactone fragrance in an amount of 10 wt% to 65 wt%, 15 wt% to 65 wt%, or 20 wt% to 65 wt%. More specifically, the fragrance composition may contain a lactone fragrance in an amount of 25 wt% to 65 wt%, 30 wt% to 65 wt%, or 35 wt% to 65 wt%. Even more specifically, the fragrance composition may contain a lactone fragrance in an amount of 40 wt% to 65 wt%, 45 wt% to 65 wt%, or 50 wt% to 65 wt%. Most specifically, the fragrance composition may contain a lactone fragrance in an amount of 55 wt% to 65 wt% or 60 wt% to 65 wt%. When the lactone fragrance is contained less than 5 wt% relative to the total amount of the fragrance, the effect of enhancing emotional quality is insignificant, whereas when the lactone fragrance is contained greater than 65 wt%, the preference to the fragrance is reduced because the fragrance character becomes simple.

The present invention can provide a fragrance composition which contains both an alcohol fragrance and a lactone fragrance. The fragrance composition which contains both fragrances may contain an alcohol fragrance in an amount of 10 wt% to 40 wt% and a lactone fragrance in an amount of 10 wt% to 50 wt%. If both the alcohol fragrance and the lactone fragrance are contained in an appropriate amount within the above ranges, the malodor suppression effect is increased. The composition which contains both the alcohol fragrance and the lactone fragrance has an increased masking suppression effect (*i.e*., so-called synergistic effect) compared to the compositions which contain either the alcohol fragrance or the lactone fragrance alone. More specifically, the composition ratio (wt%) between alcohol fragrance : lactone fragrance may be at a ratio of 10:50, 15:45, 20:40, 25:35, 30:30, 35:25, 40:20, 45:15, or 50:10.

Additionally, the present invention provides a fragrance composition in which a fragrance having a ClogP value not greater than 7 is contained in an amount of 30 wt% or greater relative to the total weight of the composition. The ClogP value refers to a logarithm value of P, where the P is the ratio between the solubility of the fragrance substance in octanol and the solubility of the fragrance substance in water.

To obtain a more excellent malodor-masking effect of alcohols or lactones, more preferably, a fragrance having the ClogP value of not greater than 7 is contained in an amount of 50 wt% or more, and most preferably 70 wt% or more, relative to the total weight of the composition. Specifically, a fragrance having the ClogP value of not greater than 7 may be contained in an amount of 50 wt%, and more specifically, in an amount of 51 wt%, 52 wt%, 53 wt%, 54 wt%, 55 wt%, 56 wt%, 57 wt%, 58 wt%, 59 wt%, 60 wt%, 61 wt%, 62 wt%, 63 wt%, 64 wt%, 65 wt%, 66 wt%, 67 wt%, 68 wt%, 69 wt%, 70 wt%, 71 wt%, 72 wt%, 73 wt%, 74 wt%, 75 wt%, 76 wt%, 77 wt%, 78 wt%, 79 wt%, 80 wt%, 81 wt%, 82 wt%, 83 wt%, 84 wt%, 85 wt%, 86 wt%, 87 wt%, 88 wt%, 89 wt%, 90 wt%, 91 wt%, 92 wt%, 93 wt%, 94 wt%, 95 wt%, 96 wt%, 97 wt%, 98 wt%, 99 wt%, or 100 wt%, relative to the total weight of the composition.

Additionally, the present invention provides a fragrance composition which can suppress malodors, in which the malodor source is at least one selected from the group consisting of isobutyric acid, isovaleric acid, caproic acid, caprylic acid, 2-nonenal, 6-heptenoic acid, and pelargonic acid. Isobutyric acid causes malodors such as discomfort, foot odor, *etc*.; isovaleric acid causes malodors such as foot odor, feces, *etc*.; and caproic acid and caprylic acid cause malodors such as scalp odor, moldy odor, oil odor, and indoor drying odor, *etc.* Further, 2-nonenal causes malodors such as oil odor, male odor, fishy odor, *etc*.; 6-heptenoic acid causes the odor of long-neglected laundry; and pelargonic acid causes malodors such as oil rancid odor and the smell of men's clothes.

In Examples of the present invention, in a case where only an alcohol fragrance was applied, the fragrance was confirmed to have an excellent malodor-control effect compared to common fragrances (Examples 1 to 3 and 5). Additionally, in a case where only a lactone fragrance was applied, the fragrance was confirmed to have an excellent malodor-control effect compared to common fragrances (Example 4). In particular, in a case where both an alcohol fragrance and a lactone fragrance were applied in combination, the malodor-masking effect of the fragrance was increased when the alcohol fragrance content was 10 wt% or greater relative to the total composition, and the malodor-masking effect of the fragrance was further increased when the lactone fragrance was added thereto (Examples 6 to 13). Further, in a case where both the alcohol fragrance and the lactone fragrance contents were each in an amount of 20 wt% to 40 wt%, it was confirmed that the masking effect of the fragrance was increased. Specifically, it was confirmed that the masking effect was significantly increased until the alcohol fragrance content reached 40 wt% (Examples 9 to 11).

Another aspect of the present invention provides a home care product for suppressing malodors including the fragrance composition.

As used herein, the term "home care product" includes products that are used at home, such as in kitchens and bathrooms, for surface cleaning or maintenance of a hygienic state (*e.g*., hard surface cleaners, manual and automatic dish care, toilet bowl cleaners and disinfectants, and kitchen detergents), and laundry care products for textile care and cleaning (*e.g*., detergents, fabric softeners, and pretreatment stain removers), air fresheners that emit aromatic compounds to remove malodors or are used as a substitute for perfumes in some cases, but the home care product is not limited thereto, and can be applied to all the home care compositions into which fragrances are added. Specifically, the home care product may include detergents, detergent additives, washing bars, fabric softeners, kitchen cleaners, wood and furniture polish, floor polish, bath tub and tile cleaners, toilet bowl cleaners, hard surface cleaners, window cleaners, antifogging agents, drain cleaners, automatic-dishwasher liquids and sheeting agents, carpet cleaners, prewash spotters, iron stain cleaners, scale removers, deodorants, and air fresheners, but the home care product is not limited thereto.

The home care product of the present invention (*e.g*., a product to be used as a laundry product) may be a textile care product including the fragrance composition and a cationic polymer, an amphoteric surfactant, or a mixture thereof, but the home care product is not limited thereto. As the cationic polymer to be used for the textile care product, at least one selected from the group consisting of cationic guar, cationic cellulose, a dimethyldiallylammonium chloride polymer, an acrylamide-dimethyldiallylammonium chloride copolymer, a PVP-dimethylaminoethyl methacrylate copolymer, an acrylic acid-dimethyldiallylammonium chloride copolymer, an acrylamide-dimethylamino ethylmethacrylate methyl chloride copolymer, a trimethylaminoethylmethacrylate polymer, *etc.* may be used, but the cationic polymer is not limited thereto. As the amphoteric surfactant to be used for the textile care product, at least one selected from the group consisting of alkylamidopropylbetaine, alkyldimethylbetaine, alkylmonoamphoacetate, cocoamphocarboxy glycinate, sacosinate, alkyl amphodiacetate, *etc.* may be used, but the amphoteric surfactant is not limited thereto.

The home care product of the present invention (*e.g*., a product to be used as a laundry product) may be a laundry detergent product including the fragrance composition and a nonionic surfactant, an anionic surfactant, or a mixture thereof, but the home care product is not limited thereto. As the nonionic surfactant to be used in the laundry detergent product, at least one selected from the group consisting of fatty acid alkyl polyoxyethylene glycol, fatty acid polyoxyethylene glycol, alkylphenyl polyoxyethylene glycol, polyoxyethylene glycol, *etc*., may be used, but the nonionic surfactant is not limited thereto. As the anionic surfactant to be used in the laundry detergent product, at least one selected from the group consisting of linear alkylbenzenesulfonate, fatty acid salt, alkylsulfonate, or alpha-olefin sulfonate, *etc.* may be used, but the anionic surfactant is not limited thereto.

The home care product of the present invention (*e.g*., a product to be used as a laundry product) requires several cleaning adjunct materials, but particular simple formulations (*e.g*., bleach additives) may require only oxygen bleaches and surfactants. The cleaning adjunct materials may include builders, enzymes, bleaches, bleach activators, catalyst materials, *etc.* Specifically, the cleaning adjunct materials may include various active ingredients or specialized materials, such as foam accelerators, foam inhibitors (antifoaming agents), dispersant polymers, color speckles, silver care agents, anti-discolorants and/or anti-corrosive agents, dyes, fillers, bactericides, alkali sources, hydrotropes, antioxidants, enzyme stabilizers, pro-fragrances, fragrances, solubilizers, carriers, processing aids, and pigments; and for liquid formulations, solvents, chelating agents, dye transfer inhibitors, dispersants, brighteners, foam inhibitors, dyes, structural elasticizers, fabric softeners, anti-abrasives, hydrotropes, processing aids, and other fabric care agents, surface and skin care agents.

The home care product of the present invention (*e.g*., air freshener) may be a volatile-type air freshener including the fragrance composition of the present invention prepared in a gel-type or solidified form, or may be a spray-type air freshener including the fragrance composition of the present invention prepared in a liquid form. Additionally, the air freshener may include the fragrance composition of the present invention in an amount of 0.001 wt% to 30 wt%, specifically 1 wt% to 30 wt%, and more specifically 1 wt% to 10 wt%, relative to the total amount of the product. When the fragrance composition is contained less than 0.001 wt%, the fragrance-emitting property is too low to savor the fragrance, whereas when the fragrance composition is contained in excess of 30 wt%, the fragrance becomes too strong to be used as an air freshener. When the fragrance composition of the present invention is used as an air freshener additive, the composition may be added as it is or mixed with a flavoring agent or any ingredient useful for the air freshener. In particular, the fragrance composition of the present invention may be mixed with at least one selected from a wide range of natural, synthetic, synthetic chemicals, natural air fresheners, flavoring materials, and natural extracts used in the field of flavoring agents and air fresheners. Additionally, the air freshener including the fragrance composition of the present invention may contain at least one component or excipient (*e.g*., a carrier material, a thickening agent, a flavor enhancer, and other aids commonly known and used in the art), which is commonly used along with flavoring agents and air fresheners.

Still another aspect of the present invention provides a personal care product for suppressing malodors including the fragrance composition.

As used herein, the term "personal care product" may include cosmetics which are applied to the bodies of humans and animals including skin, hair, scalp, and nails; hair care products; toiletries; cosmeceuticals; beauty aids; insect repellents; and personal hygiene and cleansing products, but the personal care product is not limited thereto, and can be applied to all the personal care compositions into which fragrances are added. Specifically, the personal care product may include deodorant, antiperspirants, sprays, stick and roll-on products, shaving agents, skin lotions, moisturizers, toners, bath products, cleansing agents, shampoos, conditioners, a combination of shampoo/conditioner, hair mousses, styling gels, hair sprays, hair dyes, hair colorants, hair bleaches, waving agents, hair straightening agents, nail polishes, nail polish removers, nail creams and lotions, keratin softeners, sunscreens, insect repellents, anti-aging agents, lipsticks, foundations, face powders, eyeliners, eye shadows, blushes, makeups, mascaras, moisturizing aids, body and hand aids, skin care aids, face and neck aids, tonics, dressings, hair grooming aids, aerosol fixatives, fragrance aids, aftershaves, makeup aids, soft focus supplies, night and day skincare aids, non-colored hair aids, tanning aids, synthetic and non-synthetic soap bars, hand liquids, nose strips, non-woven products for personal care, baby lotions, baby bath products and shampoos, baby conditioners, shaving aids, cucumber slices, skin pads, makeup removers, facial cleansing agents, cold creams, sunscreens, hair mousses, spritzs, paste masks and muds, face masks, cologns and lotions, hair cuticle coats, shower gels, face and body washes, personal care rinse-off agents, gels, foam bath products, scrubbing cleansers, astringents, nail conditioners, eye shadow sticks, face or eye powders, lip balms, lip glosses, hair care pump sprays and other non-aerosol sprays, hair-freeze-control gels, hair leave-in conditioners, hair pomades, hair detangling agents, hair fixatives, hair bleaches, skin lotions, pre-shaves and pre-electric shaves, anhydrous creams and lotions, oil-in-water emulsions, water-in-oil emulsions, multiple macro- and microemulsions, water-resistant creams and lotions, acne skin aids, mouthwashes, massage oils, toothpastes, clear gels and sticks, ointment bases, topical wound healing agents, aerosol talcs, barrier sprays, vitamin and anti-aging aids, herbal extract aids, bath salts, bath and body milks, hair styling aids, hair-, eye-, nail- and skin-softening products, controlled release personal care agents, hair conditioning mists, skin care moisturizing mists, skin wipes, pore skin wipes, pore cleansers, blemish reducers, skin exfoliators, skin desquamation enhancers, skin towelettes and cloths, hair removal agents, personal care lubricants, and nail coloring aids, but the personal care product is not limited thereto.

The personal care product of the present invention (*e.g*., cosmetics) may be prepared into a formulation selected from the group consisting of solutions, ointments for external use, creams, foams, toners, lotions, packs, softening water, emulsions, makeup bases, essences, soaps, liquid cleaners, bath products, sunscreen creams, sun oils, suspensions, emulsions, pastes, gels, lotions, powders, soaps, surfactant-containing cleansers, oils, powder foundations, emulsion foundations, wax foundations, patches, and sprays, but the personal care product is not limited thereto.

The cosmetics of the present invention may further include at least one cosmetically acceptable carrier that is blended in general skin cosmetics, and as a typical component, for example, oils, water, surfactants, moisturizers, lower alcohols, thickeners, chelating agents, pigments, preservatives, fragrances, *etc.* may be appropriately blended, but the cosmetically acceptable carrier is not limited thereto. The cosmetically acceptable carrier to be included in the cosmetic product of the present invention varies depending on the formulation.

When the formulation of the present invention is an ointment, paste, cream, or gel, as a carrier component, an animal oil, a vegetable oil, wax, paraffin, starch, tragacanth, a cellulose derivative, polyethylene glycol, silicone, bentonite, silica, talc, zinc oxide, or a mixture thereof may be used.

When the formulation of the present invention is powder or a spray, as a carrier component, lactose, talc, silica, aluminum hydroxide, calcium silicate, polyamide powder, or a mixture thereof may be used. In particular, when the formulation of the present invention is a spray, as a carrier component, a propellant such as chlorofluorohydrocarbon, propane/butane, and dimethyl ether may be additionally contained.

When the formulation of the present invention is a solution or emulsion, as a carrier component, a solvent, a solubilizer, or an emulsifier may be used. For example, water, ethanol, isopropanol, ethyl carbonate, ethyl acetate, benzyl alcohol, benzyl benzoate, propylene glycol, and 1,3-butylglycol oil may be used, and in particular, cottonseed oil, peanut oil, corn seed oil, olive oil, castor oil and sesame oil, glycerol aliphatic ester, polyethylene glycol or fatty acid ester of sorbitan may be used.

When the formulation of the present invention is a suspension, as a carrier component, a liquid diluent (*e.g*., water, ethanol, and propylene glycol), suspending agent (*e.g*., ethoxylated isostearyl alcohol, polyoxyethylene sorbitol ester, and polyoxyethylene sorbitan ester), microcrystalline cellulose, aluminum metahydroxide, bentonite, agar, tragacanth, *etc.* may be used.

When the formulation of the present invention is a soap, as a carrier component, an alkali metal salt of fatty acid, fatty acid hemiester salt, fatty acid protein hydrolyzate, isethionate, lanolin derivative, aliphatic alcohol, vegetable oil, glycerol, sugar, *etc.* may be used.

The cosmetics of the present invention may vary in the content of the fragrance composition according to the formulation. For example, a wash-off type cosmetic product (*e.g*., makeup removers, cleaning agents, *etc*.), in which the active ingredient stays on the skin within a short period of time, may have a relatively high concentration of the fragrance composition. In contrast, in the case of a leave-on type cosmetic product (*e.g*., emollients, emulsions, creams, essences, *etc*.), in which the active ingredient stays on the skin for a long period of time, it may be acceptable that the leave-on type cosmetic products include a lower concentration of the fragrance composition than the wash-off type cosmetic products.

The personal care product of the present invention (*e.g*., hair care products) includes hair care products, such as shampoos (including a complex shampoo such as a two-in-one conditioning shampoo), hair conditioners, after-shampoo rinse, hair dyes, setting agents, hair styling agents and hair style retaining agents (including setting aids (*e.g*., gels and sprays), grooming aids (*e.g*., pomades, conditioners, perm liquids, and relaxants)), and hair smoothing products, but the personal care product is not limited thereto. The hair care product may include the fragrance composition of the present invention and at least one selected from the group consisting of a cationic surfactant, an amphoteric surfactant, a nonionic surfactant, and a cationic polymer, but the hair care product is not limited thereto. As the cationic surfactant used in the hair care product, at least one selected from the group consisting of cetrimonium chloride, stearamidopropyl dimethylamine, dicetyl dimethyl ammonium chloride, esterquat, *etc.* may be used, but the cationic surfactant is not limited thereto. As the amphoteric surfactant used in the hair care product, at least one selected from the group consisting of cocamidopropyl betaine, cocoamphocarboxyglycinate, and cocoamphocarboxypropionate may be used, but the amphoteric surfactant is not limited thereto. As the nonionic surfactant used in the hair care product, at least one selected from the group consisting of lauric acid diethanolamide, palm oil fatty acid diethanolamide, palm oil fatty acid monoethanolamide, lauryldimethylamine oxide, palm oil alkyldimethylamine oxide, *etc.* may be used, but the nonionic surfactant is not limited thereto. As the cationic polymer used in the hair care product, at least one selected from a natural cationic polymer group (which consists of guar hydroxypropyltrimonium chloride, hydroxypropyl guar hydroxypropyltrimonium chloride, and quaternary hydroxyethyl cellulose) and a synthetic cationic polymer group (which consists of a dimethyldiallylammonium chloride polymer, an acrylamide dimethyldiallylammonium chloride copolymer, and a polyvinylpyrrolidone dimethylaminoethyl methacrylate copolymer) may be used, but the cationic polymer is not limited thereto. The hair dye may be a hair dye including the fragrance composition of the present invention and a precursor and a coupler, but is not limited thereto.

The personal care product of the present invention (*e.g*., body care products) is for bath and shower additives, formulations containing aroma- and flavor materials, hair care products, deodorants and antiperspirants, decorative preparations, and photoprotective formulations and preparations containing an active ingredient, and may be used alone or in combination with other stabilizing agents. The body care product includes body oils, body lotions, body gels, treatment creams, skin protection ointments, and shaving preparations (*e.g*., shaving foams or gels), skin powders (*e.g*., baby powder), moisturizing gels, moisturizing sprays, revitalizing body sprays, cellulite gels, and peeling agents, but the body care product is not limited thereto. Suitable bath and shower additives include shower gels, bath-salts, bubble bath agents and soaps. The preparations containing the aroma- and flavor materials include perfumes, emollients, and shaving lotions (aftershave preparations). The body care product may be in the form of a cream, ointment, paste, foam, gel, lotion, powder, makeup, spray, stick, or aerosol, but is not limited thereto. The body care product may also include a stabilizing mixture, and optionally an additional UV absorbent, steric hindered amine, complexing agent, and phenolic or non-phenolic antioxidant, but is not limited thereto.

Still another aspect of the present invention provides a method for suppressing malodors, including applying a fragrance composition for suppressing malodors to malodor sources.

As used herein, the term "fragrance composition for suppressing malodors" is as described above.

As used herein, the term "malodor source" may be at least one selected from the group consisting of isobutyric acid, isovaleric acid, caproic acid, caprylic acid, 2-nonenal, 6-heptenoic acid, and pelargonic acid, as described above, but the malodor source is not limited thereto.

### [Mode for Carrying Out the Invention]

Hereinafter, Examples and Experimental Examples of the present invention will be described in detail to facilitate understanding of the present invention. However, the Examples and Experimental Examples according to the present invention may be modified into various other forms, and the scope of the present invention should not be construed as being limited to the following Examples and Experimental Examples. The Examples and Experimental Examples are provided to more fully describe the present invention to those skilled in the art.

To demonstrate the effects of the fragrance composition of the present invention, in the following Examples and Experimental Examples, the deodorants for which immediate evaluation can be performed relatively immediate with regard to the malodors containing acids and aldehydes were used, but the scope of the present invention should not be construed as being limited to the deodorants.

### Experimental Example 1 : Preparation of fragrance bases

Fragrance Base Nos. 1 to 13 containing an alcohol fragrance and/or a lactone fragrance were prepared with each of the compositions shown in Table 3 below.

**[Table 3] Fragrance bases**

| C a t e g o r y | Fragrance Name | FB 1 | FB 2 | FB 3 | FB 4 | FB 5 | FB 6 | FB 7 | FB 8 | FB 9 | FB 10 | FB 11 | FB 12 | FB 13 |
|---|---|---|---|---|---|---|---|---|---|---|---|---|---|---|
| A l c o h o l s | Decanol | 10 | | | | | | | | | | | | |
| | Ambrinol | 10 | | | | | | | | | | | | |
| | Nerol | 10 | | | | | | | | 5 | 5 | 5 | 3.7 5 | 2.5 |
| | Styrallyl Alcohol | 10 | | | | 5 | 5 | 5 | 5 | | | | | |
| | *cis*-3-Hexen ol | 10 | | | | 5 | 5 | 5 | 5 | | | | | |
| | Anisyl Alcohol | 10 | | | | 5 | 5 | 5 | 5 | | | | | |
| | Dimetol | | 10 | | | | | | | | | | | |
| | Cinnamyl Alcohol | | 10 | | | | | | | | 5 | 5 | 3.7 5 | 2.5 |
| | Patchouli | | 10 | | | | | | | | | | | |
| | Alcohol | | | | | | | | | | | | | |
| | Timberol | | 10 | | | 5 | 5 | 5 | 5 | 5 | | | | |
| | Dihydro Myrcenol | | 10 | | | 15 | 10 | 10 | 10 | 10 | | | | |
| | Pamplefleur | | 10 | | | 15 | 10 | 10 | 10 | 10 | | | | |
| | Geraniol | | | 10 | | 5 | 5 | 5 | 5 | 5 | 5 | 5 | 3.7 5 | 2.5 |
| | Linalool | | | 10 | | | | | | | | | | |
| | Phenyl Ethyl Alcohol | | | 10 | | 5 | 5 | 5 | 5 | 5 | 5 | 5 | 3.7 5 | 2.5 |
| | Rosalva | | | 10 | | | | | | | | | | |
| | α-Terpineol | | | 10 | | | | | | | | | | |
| | Cedrol | | | 10 | | | | | | | | | | |
| L a c t o n e s | γ-Undecalac tone | | | | 20 | | 10 | | | 5 | 10 | 5 | 10 | 10 |
| | *cis*-Jasmone Lactone | | | | 20 | | | 10 | | 10 | 20 | 30 | 30 | 30 |
| | Musk Lactone | | | | 20 | | | | 10 | 5 | 10 | 5 | 5 | 10 |
| S o l v e n t | DPG | 40 | 40 | 40 | 40 | 40 | 40 | 40 | 40 | 40 | 40 | 40 | 40 | 40 |
| Total | | 10 0.0 | 100 .0 | 10 0.0 | 10 0.0 | 100 .0 | 10 0.0 | 100 .0 | 100 .0 | 100 .0 | 100 .0 | 100 .0 | 100 .0 | 10 0.0 |

| | | | | | | | | | | | | | | |
|---|---|---|---|---|---|---|---|---|---|---|---|---|---|---|
| (*FB denotes Fragrance Base.) | | | | | | | | | | | | | | |

In addition, a common fragrance (a raspberry type), which was prepared as a standard fragrance without using an alcohol fragrance or lactone fragrance, was prepared as shown in Table 4 below.

**[Table 4] A common fragrance**

| No. | Fragrance Subtance | Content |
|---|---|---|
| 1 | Nectaryl | 3.2 |
| 2 | Raspberry Ketone | 8.0 |
| 3 | Peonile | 8.8 |
| 4 | Anisyl Acetate | 12.0 |
| 5 | Isoradeine Cetone Alpha | 20.0 |
| 6 | Aldehyde C-16 | 28.0 |
| 7 | DPG | 20.0 |
| | Total | 100.0 |

### Experimental Example 2 : Preparation of fragrance compositions

Fragrance compositions for Fragrance Nos. 1 to 10 were prepared by mixing each of Fragrance Base Nos. 1 to 13 of Table 3 prepared in Experimental Example 1 above with the common fragrance of Table 4, and thus prepared fragrance compositions are shown in Table 5 below.

**[Table 5] Fragrance compositions**

| Category (wt%) | Fragrance 1 | Fragrance 2 | Fragrance 3 | Fragrance 4 | Fragrance 5 | Fragrance 6 | Fragrance 7 | Fragrance 8 | Fragrance 9 | Fragrance 10 | Fragrance 11 | Fragrance 12 | Fragrance 13 | Fragrance 14 |
|---|---|---|---|---|---|---|---|---|---|---|---|---|---|---|
| Common Fragrance | 100 | 60.0 | 60.0 | 60.0 | 60.0 | 60.0 | 60.0 | 60.0 | 60.0 | 60.0 | 60.0 | 60.0 | 60.0 | 60.0 |
| FB1 | | 40.0 | | | | | | | | | | | | |
| FB2 | | | 40.0 | | | | | | | | | | | |
| FB 3 | | | | 40. 0 | | | | | | | | | | |
| FB 4 | | | | | 40. 0 | | | | | | | | | |
| FB 5 | | | | | | 40. 0 | | | | | | | | |
| FB 6 | | | | | | | 40. 0 | | | | | | | |
| FB 7 | | | | | | | | 40. 0 | | | | | | |
| FB 8 | | | | | | | | | 40. 0 | | | | | |
| FB 9 | | | | | | | | | | 40. 0 | | | | |
| FB 10 | | | | | | | | | | | 40. 0 | | | |
| FB 11 | | | | | | | | | | | | 40. 0 | | |
| FB 12 | | | | | | | | | | | | | 40. 0 | |
| FB 13 | | | | | | | | | | | | | | 40. 0 |
| Total | 100 | 100 .0 | 100 .0 | 100 .0 | 100 .0 | 100 .0 | 100 .0 | 100 .0 | 100 .0 | 100 .0 | 100 .0 | 100 .0 | 100 .0 | 100 .0 |

| | | | | | | | | | | | | | | |
|---|---|---|---|---|---|---|---|---|---|---|---|---|---|---|
| (*FB denotes Fragrance Base.) | | | | | | | | | | | | | | |

### Experimental Example 3 : Preparation of deodorant compositions

10 Kinds of deodorant compositions (Comparative Example 2 and Examples 1 to 13) were prepared by adding each of the fragrance compositions (Fragrance Nos. 1 to 14) of Table 5 prepared in Experimental Example 2 above in an amount of 0.2 wt% to the common deodorants of Table 7 below, which are shown in Table 6 below.

As Comparative Example 1, the deodorant composition was prepared to consist of only a common deodorant.

**[Table 6] Deodorant compositions**

| | Comparative Example 1 | Comparative Example 2 | Example 1 | Example 2 | Example 3 | Example 4 | Example 5 | Example 6 | Example 7 | Example 8 | Example 9 | Example 10 | Example 11 | Example 12 | Example 13 |
|---|---|---|---|---|---|---|---|---|---|---|---|---|---|---|---|
| Fragrance 1 | | 0.2 | - | - | - | - | - | - | - | - | - | - | - | - | - |
| Fragrance 2 | - | - | 0.2 | - | - | - | - | - | - | - | - | - | - | - | - |
| Fragrance 3 | - | - | - | 0.2 | - | - | - | - | - | - | - | - | - | - | - |
| Fragrance 4 | - | - | - | - | 0.2 | - | - | - | - | - | - | - | - | - | - |
| Fragrance 5 | - | - | - | - | - | 0.2 | - | - | - | - | - | - | - | - | - |
| Fragrance 6 | - | - | - | - | - | - | 0.2 | - | - | - | - | - | - | - | - |
| Fragrance 7 | - | - | - | - | - | - | - | 0.2 | - | - | - | - | - | - | - |
| Fragrance 8 | - | - | - | - | - | - | - | - | 0.2 | - | - | - | - | - | - |
| Fragrance 9 | - | - | - | - | - | - | - | - | - | 0.2 | - | - | - | - | - |
| Fragrance 10 | | | - | - | - | - | - | - | - | - | 0.2 | | | | |
| Fragrance 11 | | | | | | | | | | | | 0.2 | | | |
| Fragrance 12 | | | | | | | | | | | | | 0.2 | | |
| Fragrance 13 | | | | | | | | | | | | | | 0.2 | |
| Fragrance 14 | | | | | | | | | | | | | | | 0.2 |
| Common Deodorant | 100 | 99.8 | 99.8 | 99.8 | 99.8 | 99.8 | 99.8 | 99.8 | 99.8 | 99.8 | 99.8 | 99.8 | 99.8 | 99.8 | 99.8 |

**[Table 7] Composition and contents of common deodorants**

| | Characteristics | Components | Content (wt%) |
|---|---|---|---|
| 1 | Deodorant | Cyclodextrin (5.5 DOS HPBCD) | 1.00 |
| 2 | Surfactant | Silwet L-77 | 0.20 |
| 3 | Surfactant | POE-40 (HCO) | 0.10 |
| 4 | Stabilizer | Sodium polyacrylate (2,500 MW) | 0.10 |
| 5 | Solvent | Ethanol | 3.00 |
| 6 | Preservative | Preservative (ppm) | 3.00 |
| 7 | Fragrance | Perfume | 0.20 |
| 8 | Solvent | Distilled water (DW) | To 100.0 |

### Experimental Example 4 : Sensory evaluation of malodors

For sensory evaluation of malodors with respect to the 15 kinds of deodorant compositions (Comparative Examples 1 and 2 and Examples 1 to 13) prepared in Experimental Example 3 above, malodor sources shown in Table 8 below were standardized and used.

**[Table 8] Standard composition of malodor sources**

| | Name of Malodor Component | Evaluation | Content (wt%) |
|---|---|---|---|
| 1 | Isobutyric Acid | Discomfort, Foot odor | 0.0200 |
| 2 | Isovaleric Acid | Foot odor, Stool odor | 0.0005 |
| 3 | Caproic Acid | Scalp odor, Moldy odor, Oil odor, Indoor drying odor | 0.0600 |
| 4 | Caprylic acid | Scalp odor, Moldy odor, Oil odor, Indoor drying odor | 0.0600 |
| 5 | 2-Nonenal | Oil odor, Male odor, Fishy Odor | 0.0005 |
| 6 | 6-Heptenoic Acid | Long-neglected laundry odor | 0.4000 |
| 7 | Pelargonic Acid | Oil rancid smell, Man's clothes smell | 0.0800 |
| | DPG | | to 100.0 |

Then, the evaluation for each odor was performed by general consumers as shown below, in which the 7 kinds of raw materials of Table 8 above were used as the malodor standard.

A test cotton cloth (10 cm × 10 cm) was placed in each 2 L beaker, and the standard malodor source of Table 8 (diluted in 1% ethanol) was sprayed 4 times thereon, and the top portion of the test cotton cloth was covered with a foil. After placing each beaker at room temperature for 5 minutes, each of the deodorant compositions of Comparative Examples 1, 2 and Examples 1 to 13, which were prepared in Experimental Example 3 above, was sprayed twice onto the test cotton cloth in each beaker, and the top portion of the test cotton cloth was covered with a foil and the cotton cloth was placed again at room temperature for 10 minutes. After 10 minutes, an aperture of 1 cm in diameter was made in the foil of each beaker to smell and evaluate each malodor.

The sensory evaluation was performed by 20 adult men and women between the ages of 20s and 40s, and the criteria for sensory evaluation are as follows.

### - Criteria for sensory evaluation -

1 point: no malodor
2 points: a mild malodor
3 points: a bit strong malodor
4 points: a strong malodor
5 points: a very strong malodor

The malodor evaluation results are shown in Table 9 below.

**[Table 9]**

| | Co mp ara tiv e Ex am ple 1 | Co mp ara tiv e Ex am ple 2 | Ex am ple 1 | Ex am ple 2 | Ex am ple 3 | Ex am ple 4 | Ex am ple 5 | Ex am ple 6 | Ex am ple 7 | Ex am ple 8 | Ex am ple 9 | Ex am ple 10 | Ex am ple 11 | Ex am ple 12 | Ex am ple 13 |
|---|---|---|---|---|---|---|---|---|---|---|---|---|---|---|---|
| M ea n | 4.7 0 | 3.6 0 | 2.7 0 | 2.6 0 | 2.7 0 | 2.2 0 | 2.5 5 | 1.8 5 | 1.9 0 | 1.6 5 | 1.3 5 | 1.5 0 | 1.5 0 | 1.6 5 | 1.7 0 |
| St an da | 0.4 6 | 0.5 8 | 0.7 9 | 0.7 2 | 0.8 0 | 0.6 8 | 0.7 3 | 0.8 1 | 0.7 8 | 0.7 3 | 0.5 0 | 0.6 9 | 0.6 1 | 0.6 7 | 0.7 3 |
| rd De vi ati on | | | | | | | | | | | | | | | |

As shown in Table 9 above, it was confirmed that there was a significant difference between the malodor evaluation result for the deodorant composition of Comparative Example 1, in which the deodorant composition was treated with no fragrance, and that for the deodorant composition of Comparative Example 2, in which the deodorant composition was treated with only the common fragrance. Further, it was confirmed that common fragrance was able to mask malodors to a certain degree, but the masking effect of the common fragrance was determined not to be effective in removing malodors.

Examples 1 to 3, and 5 describe cases where each alcohol fragrance alone was applied to the common fragrance, and it was confirmed that these alcohol fragrances were able to effectively suppress malodors compared to the common fragrance. Example 4 describes a case where each lactone fragrance alone was applied to the common fragrance, and it was confirmed that the evaluation results of these lactone fragrances regarding the malodor suppression effect were lower than those of the alcohol fragrances, but these lactone fragrances showed an excellent ability to suppress malodors compared with the deodorant composition of Comparative Example 2 in which the deodorant composition was treated with only the common fragrance.

Examples 6 to 13 describe cases of deodorant compositions where an alcohol fragrance and a lactone fragrance were applied in combination and which were shown to be more effective compared to cases of deodorant compositions where either an alcohol fragrance or a lactone fragrance alone was applied to the common fragrance. In particular, reviewing the malodor evaluation result of the deodorant composition of Example 9, it was confirmed that the masking effect of the deodorant composition was increased in a case where an alcohol fragrance and a lactone fragrance were mixed together, and the masking effect of the deodorant composition was further increased when a lactone fragrance was added thereto.

Accordingly, from the foregoing, it was confirmed that the fragrance composition which contains an alcohol fragrance, a lactone fragrance, or both has an excellent malodor suppressing effect and thus can be used for suppression of malodors, and the fragrance composition can also be used for home care products and personal care products for suppressing malodors.

## Claims

1. A fragrance composition for suppressing malodor comprising an alcohol fragrance, a lactone fragrance, or both.

2. The fragrance composition of claim 1, wherein the alcohol fragrance comprises at least one selected from the group consisting of decanol, ambrinol, dihydro myrcenol, nerol, Pamplefleur, geraniol, linalool, phenyl ethyl alcohol, rosalva, tetrahydrolinalool, α-terpineol, styrallyl alcohol, *cis*-3-hexenol, anisyl alcohol, Dimetol, cinnamyl alcohol, cedrol, patchouli alcohol, and Timberol.

3. The fragrance composition of claim 1, wherein the lactone fragrance comprises at least one selected from the group consisting of γ-undecalactone, *cis*-jasmone lactone, and musk lactone.

4. The fragrance composition of any one of claims 1 to 3, wherein the composition comprises the alcohol fragrance in an amount of 10 wt% to 65 wt% relative to the total weight of the composition.

5. The fragrance composition of any one of claims 1 to 3, wherein the composition comprises the lactone fragrance in an amount of 5 wt% to 65 wt% relative to the total weight of the composition.

6. The fragrance composition of any one of claims 1 to 3, wherein the composition suppresses the malodor which comprises, as a malodor source, at least one selected from the group consisting of isobutyric acid, isovaleric acid, caproic acid, caprylic acid, 2-nonenal, 6-heptenoic acid, and pelargonic acid.

7. A home care product for suppressing malodor, comprising the fragrance composition of any one of claims 1 to 3.

8. A personal care product for suppressing malodor, comprising the fragrance composition of any one of claims 1 to 3.

9. A method for suppressing malodor, comprising applying the fragrance composition of any one of claims 1 to 3 to a malodor source.
